# EUROPEAN PATENT APPLICATION

(11) **EP 1 820 466 A1**
(43) Date of publication of application: **22.08.2007**
(21) Application number: 07002138.1
(22) Date of filing: 31.01.2007
(51) Int. Cl.: A61C 5/08, A61C 5/10

(54) **Dental prosthesis, designing method thereof, and production method thereof**

(30) Priority: 16.02.2006 JP 2006039715
(71) Applicant: GC Corporation, Itabashi-ku Tokyo 174-8585 (JP)
(72) Inventor: Yamamoto, Shogo, Shinjuku-ku Tokyo (JP); Matsuda, Yoshinori, Itabashi-ku Tokyo 174-8585 (JP)
(74) Representative: Müller-Boré & Partner Patentanwälte

(57) **Abstract**

An objective of the present invention is to provide a dental prosthesis which is to be mounted in a cavity or covered on an abutment tooth, where the dental prosthesis can be accurately fixed at the cavity or the abutment tooth, and to provide a method of designing the dental prosthesis using a computer, and a method of producing the dental prosthesis designed by the method of designing by a machine using a CAD/CAM system or a CAD system. The dental prosthesis has a constitution in which a cement space C is kept on a whole face opposite to a cavity or an abutment tooth except a plurality of projection parts P contacted with the cavity or the abutment tooth and a portion near a margin line if necessary. The dental prosthesis which is to be covered on the abutment tooth is designed by measuring a gypsum model produced based on an impression taken from an oral cavity of a patient so as to obtain three-dimensional measuring data, providing a plurality of projection parts P contacted with the abutment tooth and a portion near a margin line if necessary based on the obtained three-dimensional measuring data, and setting a cement space C on a whole face opposite to the cavity or the abutment tooth except the contacting portion.

## Description

The present invention relates to a dental prosthesis which is to be mounted in a cavity or covered on an abutment tooth, where the dental prosthesis can be accurately fixed in the cavity or at the abutment tooth. Further, the present invention also relates to: a method of designing the dental prosthesis using a computer; a method of producing the dental prosthesis designed by the method of designing a dental prosthesis by a cutting and grinding machine using a CAD/CAM system; and a method of producing a dental prosthesis designed by the method of designing a dental prosthesis in which a photo-curable resin is photosensitively cured for every thin section of the dental prosthesis by laser irradiation from an optical shaping apparatus using a CAD system.

A dental prosthesis such as an inlay mounted in a cavity, a crown or bridge covered on an abutment tooth or the like is generally fixed in the cavity or at the abutment tooth through a dental cement. Thus, a face opposite to the cavity or the abutment tooth is necessarily formed to have a space (referred to as a cement space, hereinafter) between the opposite face and the cavity or the abutment tooth, where the space corresponds to the thickness of the dental cement.

This cement space is formed on a whole face opposite to the cavity or the abutment tooth, or a whole face opposite to the cavity or the abutment tooth except a portion having the slight length from a margin line. Thus, when the dental prosthesis is mounted in a cavity of a tooth of a patient or is covered on an abutment tooth of a patient, there is a problem that the dental prosthesis is not accurately fixed in a desirable state but fixed in an inclined state, a lifted state, or a slightly rotated state due to having a degree of freedom corresponding to the thickness of the cement space.

Generally, the dental prosthesis gives large uncomfortable feeling to a patient when the position of an occlusion face is only slightly high. Thus, when the dental prosthesis is mounted in a cavity of a tooth of a patient or covered on an abutment tooth of a patient, it is remarkably important to fix the dental prosthesis at the tooth or the abutment tooth of a patient in the desirable state.

However, an operation for fixing a conventional dental prosthesis at the tooth or the abutment tooth of a patient in the desirable state depends on a skill of a dentist. So, it has not been considered to realize the technique for fixing the dental prosthesis at the tooth or the abutment tooth of a patient in the desirable state without depending on a technique of a dentist.

In recent years, as a method for stably providing many dental prosthesis having a fixed quality in a short time, a CAD/CAM system has been widely used, where the CAD/CAM system is for designing a dental prosthesis such as an inlay, a crown, a bridge or the like on a screen using a computer, and producing a dental prosthesis by cutting and grinding. As for the designing and producing system of dental prosthesis using the CAD/CAM system, for example, Cerec System (a system produced by Siemens Corporation, Germany) has been widely used.

Further, the following method of producing a dental prosthesis has also been used. It is a method for designing a dental prosthesis such as an inlay, a crown, a bridge or the like on a screen using a computer, and photosensitively curing a photo-curable resin is carried out for every thin section of the dental prosthesis by laser irradiation from an optical shaping apparatus using a CAD system (for example, refer to Japanese Patent Application Laid Open No. 2003-145629).

In the CAD/CAM system and the CAD system, an objective dental prosthesis is designed using a computer. In the case of the CAD/CAM system, a block state material such as a resin hardened body, a ceramic sintered body, a metal body or the like is set on an automatic cutting and grinding machine, and cut and grinded based on the designing data so as to produce an objective dental prosthesis. In the case of the CAD system, the photo-curable resin is photosensitively cured for every thin section of a dental prosthesis by laser irradiation from an optical shaping apparatus based on the designing data, so as to produce an objective dental prosthesis.

In such the CAD/CAM system or the CAD system in a dental field, an impression of a shape in an oral cavity (a teeth shape or a tooth line shape) of a patient is taken using a dental impression material at first, so as to produce a gypsum model from the impression. In the case of the dental prosthesis which is to be mounted in a cavity, a secondary gypsum model or a wax model is produced from the gypsum model. Further, in the case of the dental prosthesis which is to be covered on an abutment, the gypsum model is used as it is. The three-dimensional coordinate information of these gypsum models are measured using a laser measuring instrument or the like, and three-dimensional coordinate information of the tooth line shape on the gypsum model side and the tooth line shape on the opposite teeth side are also measured.

Then, a dental prosthesis is designed using a computer based on the obtained measured data. In the case that the dental prosthesis is an inlay, the following operations are carried out: A designing operation for determining a margin line in the secondary gypsum model or the wax model; a designing operation for making a whole face opposite to a cavity of a tooth or a whole face opposite to a cavity of a tooth except a portion within a specified distance from a margin line to be an external shape, in which the thickness corresponding to a cement space is reduced from the secondary gypsum model or the wax model; and a designing operation of a shape on the occlusion face side.

Further, in the case that the dental prosthesis is a crown or a bridge, the following operations are carried out: A designing operation for determining a margin line in the gypsum model; a designing operation for making a whole face opposite to a cavity or a whole face opposite to a cavity of a tooth except a portion within a specified distance from a margin line to be an inner face shape, in which the corresponding to a cement space is added to the gypsum model; and a designing operation of shape of a portion exposed in an oral cavity (for example, refer to Japanese Patent Application Laid Open No. 2002-224142, and Fig. 13 of Domestic re-publication of PCT international application No. WO 01/091664).

The design of the dental prosthesis is completed as described above. Then, in the case of using the CAD/CAM system, production of a dental prosthesis is completed by selecting a block material to be machined, and cutting and grinding a block for an objective dental prosthesis by a cutting and grinding machine using the CAD/CAM system, and subjecting it to a trimming. In the case of using the CAD system, production of a dental prosthesis is completed by irradiating laser from an optical shaping apparatus to a photo-curable resin using the CAD system, photosensitively curing the photo-curable resin for every thin section of a dental prosthesis based on the designing data, and taking out the photosensitively cured photo-curable resin.

However, as for the dental prosthesis produced by the CAD/CAM system or the CAD system, the cement space is formed on the whole face opposite to a cavity or an abutment tooth or the whole face except a portion within a specified distance from a margin line. Thus, when the dental prosthesis is mounted in a cavity of a tooth of a patient or covered on an abutment tooth of a patient, there is a problem that the dental prosthesis is not fixed in a desirable state but fixed in an inclined state, a lifted state or a slightly rotated state due to having much freedom corresponding to the thickness of the cement space.

The present invention solves the problem at the time of mounting a dental prosthesis in a cavity of a tooth of a patient or covering it on an abutment tooth of a patient, where the problem is that a dental prosthesis is not fixed in a desirable state but fixed in an inclined state, a lifted state or a slightly rotated state due to having a degree of freedom corresponding to the thickness of a cement space. An objective of the present invention is to provide a dental prosthesis capable of being accurately fixed at a cavity or an abutment tooth; a method of designing the dental prosthesis using a computer; a method of producing the dental prosthesis designed by the method of designing a dental prosthesis by a cutting and grinding machine using a CAD/CAM system; and a method of producing a dental prosthesis designed by the method of designing a dental prosthesis in which a photo-curable resin is photosensitively cured for every thin section of the dental prosthesis by laser irradiation from an optical shaping apparatus using a CAD system.

The earnest work was carried out in order to solve the above-mentioned problems and, as a result of this, present inventors found out that the reason of causing the problem at the time of mounting a dental prosthesis in a cavity of a tooth of a patient or covering it on an abutment tooth of a patient is due to having a degree of freedom corresponding to the thickness of a cement space, where the problem is that a dental prosthesis is not fixed in a desirable state but fixed in an inclined state, a lifted state or a slightly rotated state. Further, the followings were found out to complete the present invention. Without forming a cement space on the whole face opposite to the cavity or the abutment tooth of the dental prosthesis, a plurality of projection parts which are contacted with the cavity or the abutment tooth is pre-formed on the face opposite to the cavity or the abutment tooth of the dental prosthesis, and a cement space is formed on the whole face opposite to the cavity or the abutment tooth except the projection parts, or the projection parts and a portion within a distance of 0.1 to 1 mm from a margin line. As a result of this, in the case that the dental prosthesis is fixed at the cavity or the abutment tooth using a dental cement, as the dental prosthesis is mounted in the cavity of a tooth of a patient or covered on the abutment of a patient so as to contact the projection parts to the cavity or the abutment tooth, the positioning relation between the dental prosthesis and the cavity or the abutment tooth is uniquely determined by the projection parts. Therefore, when the dental prosthesis is mounted in a cavity of a tooth of a patient or covered on an abutment tooth of a patient, there occurs no problem that the dental prosthesis is not fixed in a desirable state but fixed in an inclined state, a lifted state or a slightly rotated state.

Further, the followings were found out. In such a dental prosthesis, when the maximum thickness of the cement space is 15 to 100 µm, a position of the margin line can be accurately fixed at the cavity or the abutment tooth with the sufficient strength. Further, if the projection part is formed to have a streak shape being approximately parallel to a tooth axis, it can be easily carry out to operate for restoring the dental prosthesis to be properly fitted by grinding and slightly adjusting the projection part, when the dental prosthesis is found to be not fittable by pre-mounting before being fixed at the cavity or the abutment tooth. Further, it is preferable to provide a projection part contacted with a bottom face of the cavity in the case of the dental prosthesis which is to be mounted in the cavity, and a projection part contacted with a top part of the abutment tooth in the case of the dental prosthesis which is to be covered on the abutment tooth. By providing these projection parts, when the dental prosthesis is found to be not fittable by pre-mounting before being fixed at the cavity or the abutment tooth of a patient, it can be easily carried out to operate for restoring the dental prosthesis to be properly fitted by grinding and slightly adjusting the projection part.

Further, the followings were also found out. When such the dental prosthesis is designed by a computer, a gypsum model is produced based on an impression taken from the oral cavity of a patient. In the case of the dental prosthesis which is to be mounted in a cavity, a secondary gypsum model or a wax model is further produced based on the gypsum model so as to obtain three-dimensional measuring data from it, and in the case of the dental prosthesis which is to be covered on an abutment tooth, three-dimensional measuring data is obtained from the gypsum model. The dental prosthesis which is to be mounted in the cavity or covered on the abutment tooth can be designed based on these three-dimensional measuring data. When such a dental prosthesis is designed, it is preferable that a plurality of projection parts contacted with the cavity or the abutment tooth is provided, and a portion within the distance of 0.1 to 1 mm from the margin line is made to contact to the cavity or the abutment tooth. Further, the cement space is formed on the whole face opposite to the cavity or the abutment tooth except the projection parts, or on the whole face opposite to the cavity or the abutment tooth except the projection parts and the portion within the distance of 0.1 to 1 mm from the margin line. Such a dental prosthesis, which is designed by a computer as abovementioned, is produced by cutting and grinding a block for a dental prosthesis by a cutting and grinding machine using the CAD/CAM system, or is produced by photosensitively curing the photo-curable resin by laser irradiation from an optical shaping apparatus using the CAD system. By this method, the dental prosthesis can be easily produced.

As for the dental prosthesis according to the present invention, a plurality of the projection parts contacted with the cavity or the abutment tooth is provided on the face opposite to the cavity or the abutment tooth. Thereby, when the dental prosthesis is mounted in the cavity of a tooth of a patient or covered on the abutment tooth of a patient so as to contact the projection parts to the cavity or the abutment tooth, a positioning relation between the dental prosthesis and the cavity or the abutment tooth is uniquely determined by the projection parts. Thus, when the dental prosthesis is mounted in the cavity of a tooth of a patient or covered on the abutment tooth of a patient, there is no problem that the dental prosthesis is not fixed in the desired state but fixed in the inclined state, the lifted state or the slightly rotated state. Further, this effect is more remarkable if the portion within the distance of 0.1 to 1 mm from the margin line is formed so as to contact to the cavity or the abutment tooth.

Further, in such a dental prosthesis, when the maximum thickness of the cement space is 15 to 100 µm, a position of the margin line can be accurately fixed at the cavity or the abutment tooth with the sufficient strength. Further, when the projection part is formed to have a streak shape being approximately parallel to a tooth axis, it can be easily carried out to operate for restoring the dental prosthesis to be properly fittable by grinding and finely adjusting the projection part, when the dental prosthesis is found to be not fittable by pre-mounting before fixed at the cavity of the abutment tooth. In addition to the projection part having a streak shape, it is further preferable to provide the projection part contacted with the bottom face of the cavity in the case of the dental prosthesis which is to be mounted in the cavity, and the projection part contacted with the top part of the abutment tooth in the case of the dental prosthesis which is to be covered on the abutment tooth. By providing these projection parts, when the dental prosthesis is found to be not fittable by pre-mounting before fixed at the cavity or the abutment tooth of a patient, it can be easily carried out to operate for restoring the dental prosthesis to be properly fittable by grinding and finely adjusting the projection part.

Further, when such a dental prosthesis is designed by a computer, the gypsum model is produced based on the impression taken from the oral cavity of a patient, or the secondary gypsum model or the wax model is further produced based on the gypsum model. Then, the three-dimensional measuring data is obtained from those. The dental prosthesis which is to be mounted in the cavity or covered on the abutment tooth can be designed based on such a three-dimensional measuring data. When such a dental prosthesis is designed it is preferable that a plurality of the projection parts contacted with the cavity or the abutment tooth is provided, and the portion within the distance of 0.1 to 1 mm from the margin line is also made to be contacted with the cavity or the abutment tooth. Then, the cement space is formed on the whole face opposite to the cavity or the abutment tooth except the projection parts, or on the whole face opposite to the cavity or the abutment tooth except the projection parts and the portion within the distance of 0.1 to 1 mm from the margin line. Such a dental prosthesis, which is designed by a computer as abovementioned, is produced by cutting and grinding the block for a dental prosthesis by a cutting and grinding machine using the CAD/CAM system, or is produced by photosensitively curing the photo-curable resin by laser irradiation from an optical shaping apparatus using the CAD system. By this method, the dental prosthesis can be easily produced.

Fig. 1 is an enlarged perspective view illustrating an example of an inlay which is a dental prosthesis according to the present invention, where projection parts approximately parallel to a tooth axis and a projection part contacted with a bottom face of a cavity are provided.

Fig. 2 is a cross sectional explanation view illustrating a state that the dental prosthesis illustrated in Fig. 1 is pre-mounted in a cavity of a tooth of a patient.

Fig. 3 is an enlarged perspective view illustrating an example of a crown which is a dental prosthesis according to the present invention, where projectionparts approximately parallel to a tooth axis and a projection part contacted with a top face of an abutment tooth are provided.

Fig. 4 is cross sectional explanation view illustrating a state that the dental prosthesis illustrated in Fig. 3 is pre-mounted on an abutment tooth of a patient.

Fig. 5 is an explanation view illustrating a state that a wax model produced based on an impression taken from an oral cavity of a patient is measured so as to obtain three-dimensional data in a method of designing a dental prosthesis according to the present invention, where the dental prosthesis is an inlay.

Fig. 6 is an explanation view illustrating a state that a gypsum model of an abutment tooth, which is produced based on an impression taken from an oral cavity of a patient, is measured so as to obtain three-dimensional data in a method of designing a dental prosthesis according to the present invention, where the dental prosthesis is a crown.

Hereinafter, a dental prosthesis according to the present invention, and a method of designing and producing thereof are particularly described with drawings.

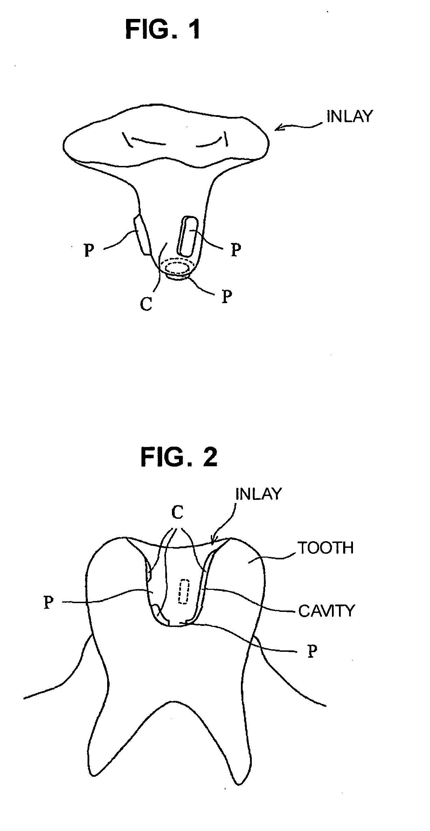

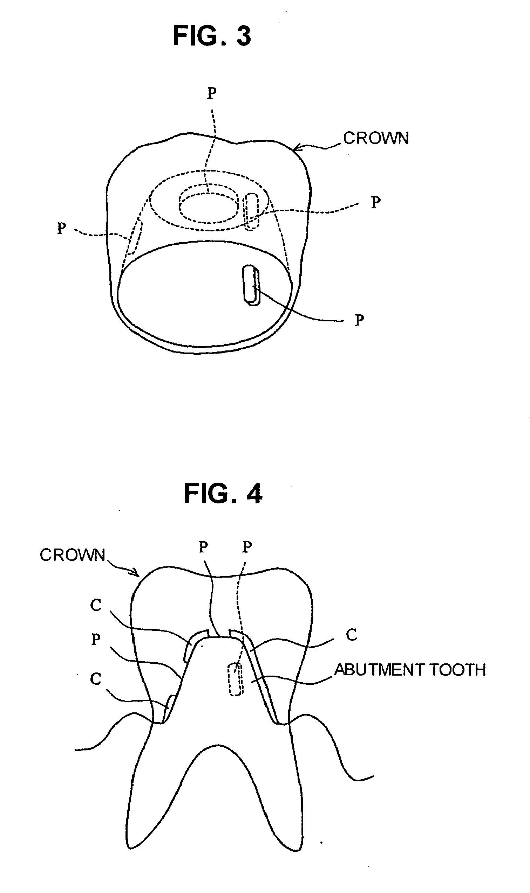

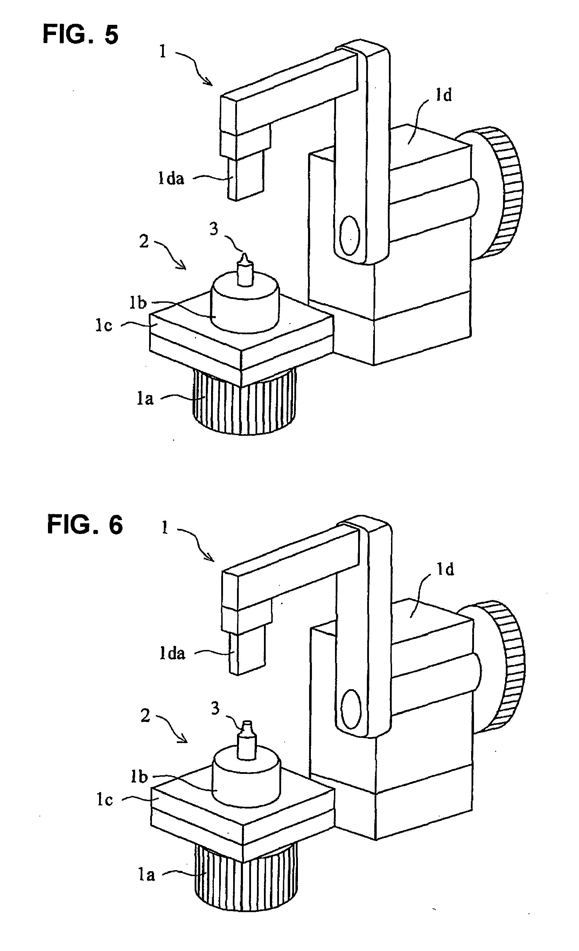

Fig. 1 is an enlarged perspective view illustrating an example of an inlay which is a dental prosthesis according to the present invention, where projection parts approximately parallel to a tooth axis and a projection part contacted with a bottom face of a cavity are provided. Fig. 2 is a cross sectional explanation view illustrating a state that the dental prosthesis illustrated in Fig. 1 is pre-mounted in a cavity of a tooth of a patient. Fig. 3 is an enlarged perspective view illustrating an example of a crown which is a dental prosthesis according to the present invention, where projection parts approximately parallel to a tooth axis and a projection part contacted with a top face of an abutment tooth are provided. Fig. 4 is cross sectional explanation view illustrating a state that the dental prosthesis illustrated in Fig. 3 is pre-mounted on an abutment tooth of a patient. Fig. 5 is an explanation view illustrating a state that a wax model produced based on an impression taken from an oral cavity of a patient is measured so as to obtain three-dimensional data in a method of designing a dental prosthesis according to the present invention, where the dental prosthesis is an inlay. Fig. 6 is an explanation view illustrating a state that a gypsum model of an abutment tooth, which is produced based on an impression taken from an oral cavity of a patient, is measured so as to obtain three-dimensional data in a method of designing a dental prosthesis according to the present invention, where the dental prosthesis is a crown.

A dental prosthesis according to the present invention is described at first. The dental prosthesis according to the present invention is a dental prosthesis which is to be mounted in a cavity or covered on an abutment tooth. In the dental prosthesis, cement space C is kept on the whole face opposite to the cavity or the abutment tooth except a plurality of projection parts P contacted with the cavity or the abutment tooth or except the projection parts P and a portion within a distance of 0.1 to 1 mm from a margin line.

That is, when the dental prosthesis is mounted in the cavity, the dental prosthesis is an inlay as illustrated in Fig. 1. The dental prosthesis includes a face for forming an occlusion face, and a foot shaped portion which is formed on the opposite side to the face for forming this occlusion face and mounted in the cavity. In an example illustrated in Fig. 2, the foot shaped portion has a plurality of projection parts P on the side face thereof, which is contacted with the side face of the cavity, a portion contacted with the side face of an opening part of the cavity at a portion within a distance of 0.1 to 1 mm from a margin line, and a projection part contacted with a bottom face of the cavity. The cement space C is kept on the whole face opposite to the cavity except the projection parts P and the portion within the distance of 0.1 to 1 mm from the margin line.

Further, when the dental prosthesis is covered on the abutment tooth, the dental prosthesis is a crown or a bridge (the illustrated example is a crown) as illustrated in Fig. 3. The dental prosthesis includes a portion for forming an external shape of a tooth exposed in an oral cavity, and a portion covered on the abutment tooth at the inner side of the portion for forming an external shape of a tooth. In an example illustrated in Fig. 4, the cement space C is kept on the whole face of a recessed portion covered on the abutment tooth except a plurality of projection parts P contacted with the abutment tooth and a projection part P contacted with a top part of the abutment tooth.

In order to design and produce such a dental prosthesis, there are two cases using a lost wax method and using a computer, which have been conventionally used. At first, a case of using a lost wax method is described.

In the case of using the lost wax method, an impression of a shape in an oral cavity (a tooth shape or a tooth line shape) of a patient is taken using a dental impression material so as to produce a gypsum model of the shape in an oral cavity based on the impression.

Then, when a dental prosthesis mounted which is to be in a cavity of a tooth is produced, the dental prosthesis is produced by the steps of: putting a wax into a cavity formed at the gypsum model; removing the wax model from the gypsum model after the wax is cured; determining a margin line; forming a shape of a portion (an occlusion face) exposed in the oral cavity; removing the face of the wax model, which is opposite to an inner face of the cavity, to have the removed thickness corresponding to a cement space C so as to keep the cement space C, where the face is removed except a portion in which projection parts P are provided and a portion within the distance of 0.1 to 1 mm from the margin line if necessary; embedding the wax model in a gypsum for embedding; heating it so as to burn the wax model; putting a melted dental alloy for casting into a space formed by burning of the wax model; taking the solidified dental alloy for casting from the gypsum for embedding after solidifying of the dental alloy for casting; and trimming it. As for the dental prosthesis, a ceramic material is built to be baked on the portion exposed in the oral cavity, or a resin for facing a crown is built to be cured on the portion exposed in the oral cavity, if necessary.

Further, when a dental prosthesis which is to be covered on an abutment tooth is produced, the dental prosthesis is produced by the steps of: determining a margin line on the gypsum model; coating a coating liquid for forming a cement space (for example, the product name of "Share Spacer" produced by GC Corporation) onto the face of the gypsum model so as to keep a cement space C, where the coating liquid is coated onto the face except a portion in which projection parts are provided and a portion within the distance of 0.1 to 1 mm from the margin line if necessary; building a wax over the coating while adjusting the margin line so as to form a shape of a portion exposed in an oral cavity; embedding the wax model taken out from the gypsum model in a gypsum for embedding; heating it so as to burn the wax model; putting a melted dental alloy for casting into a space formed by burning of the wax model; taking the solidified dental alloy for casting from the gypsum for embedding after solidifying of the dental alloy for casting; and trimming it. As for the dental prosthesis, a ceramic material is built to be baked on the portion exposed in the oral cavity, or a resin for facing a crown is built to be cured on the portion exposed in the oral cavity, if necessary.

Further, a case of designing and producing such a dental prosthesis using a computer will be described. A gypsum model of a shape in an oral cavity is produced based on the impression taken from the inside of an oral cavity of a patient. In the case of a dental prosthesis which is to be mounted in a cavity, a secondary gypsum model or a wax model having the shape of the inside of the cavity is produced based on the gypsum model so as to obtain three-dimensional measuring data. In the case of a dental prosthesis which is to be covered on an abutment tooth, three-dimensional measuring data is obtained from the gypsum model. The both of three-dimensional measuring data are obtained using a three-dimensional coordinate measuring device.

The three-dimensional measuring data is obtained by using a three-dimensional coordinate measuring device 1, which includes: a rotary table 1a having an axial center of a rotary axis forming a Z-axis; a XY table 1c being provided on the rotary table 1a and movable in the X axis and Y axis directions, and having a mounting stand 1b fixed at an upper part thereof for providing a measured object mounting tool 2; and a measuring part 1d for measuring three-dimensional coordinate of the shape of the measured object mounted on the measured object mounting tool 2 on the mounting stand 1b. The three-dimensional coordinate is measured by one laser sensor 1da . The laser sensor 1da can be rotated and moved on the same plane including the Z axis around a desired point on the Z axis, and can be moved in the Z axis direction. In the case of the dental prosthesis which is to be mounted in an cavity, a secondary gypsum model or a wax model 3, which is produced from the gypsum model, is mounted on the measured object mounting tool 2 on the mounting stand 1b as illustrated in Fig. 5, and in the case of the dental prosthesis which is to be covered on the abutment tooth, the gypsummodel is mounted on the measured object mounting tool 2 on the mounting stand 1b as illustrated in Fig. 6. After that, the rotary table 1a is rotated around the Z axis, and the measuring part 1d is rotated and moved on the same plane including the Z axis, so as to irradiate laser from the laser sensor 1da toward the secondary gypsum model or the wax model, or the gypsum model 3. Thereby, the three-dimensional coordinates of the secondary gypsum model or the wax model 3, or the gypsum model 3 can be measured and obtained.

When the three-dimensional measuring data of the secondary gypsum model or the wax model, or the gypsum model are obtained, the dental prosthesis which is to be mounted in the cavity or covered on the abutment tooth is designed based on the three-dimensional measuring data. When the dental prosthesis is an inlay mounted in the cavity of a tooth, the designing is carried out based on the measuring data by: using a drawing display device such as a CRT screen of a computer or the like; transforming an outline of the margin line of the dental prosthesis based on a three-dimensional graphic of the shape of the secondary gypsum model or the wax model, which is displayed on the drawing display device; and adjusting the margin line of the dental prosthesis to meet the margin line of abutment tooth. After that, the following two designing operations are further carried out. One designing operation is carried out for the face opposite to the cavity of a tooth to have an outer face shape, which is formed by reducing the thickness corresponding to the cement space C from the whole face opposite to the cavity of a tooth, where the whole face starts from a position at the desired distance, which is preferably 0.1 to 1 mm, from the margin line except the projection parts P contacted with the cavity of a tooth, and the maximum of the thickness is preferably within the range of 15 to 100 µm. Another designing operation is carried out for a shape at the occlusion face side based on the tooth line shape at the opposite tooth side using the three-dimensional graphic of the shape in the oral cavity.

Further, when the dental prosthesis is a crown or a bridge mounted on the abutment tooth, the designing is carried out based on the obtained measuring data by: using a drawing display device such as a CRT screen of a computer or the like; transforming an outline of the margin line of the dental prosthesis based on a three-dimensional graphic of the shape of the gypsum model, which is displayed on the drawing display device, and adjusting the margin line of the dental prosthesis to meet the margin line of abutment tooth. After that, the following two designing operations are further carried out. One designing operation is carried out for a face opposite to the abutment tooth to have an inner face shape, which is formed by adding the thickness corresponding to the cement space C to the whole face opposite to the abutment tooth, where the whole face starts from a position at the desired distance, which is preferably 0.1 to 1 mm, from the margin line except the projection parts P contacted with the abutment tooth, and the maximum of the thickness is preferably within the range of 15 to 100 µm. Another designing operation is carried out for an outer face shape exposed in the oral cavity using three-dimensional graphics of the tooth line shape at the abutment tooth side and the tooth line shape at the opposite tooth side.

In such a designing, a shape of the projection part P is not especially restricted if it is within a range in which a function of the cement space C is not restricted. For example, the shape is a streak shape, a pillar shape, a Mt. Fuji shape or the like. However, when the dental prosthesis is produced by cutting and grinding a block for a dental prosthesis by a cutting and grinding machine using the CAD/CAM system based on the designing, it is preferable that the projection part P has the streak shape being approximately parallel to the tooth axis which corresponds to the cutting direction if the cutting and grinding machine is a type using a cutting bar or the like. Because, by having such a shape, the cutting can be easily carried out. Further, it is preferable that the number of projection parts P is 3 or more, and especially, 3 projection parts are preferable since fine adjustment for a proper angle of the dental prosthesis can be easily made. Further, in the case of the dental prosthesis which is to be mounted in the cavity, a projection part contacted with a bottom face of the cavity is further provided, and in the case of the dental prosthesis which is to be covered on the abutment tooth, a projection part contacted with a top part of the abutment tooth is further provided. By providing these projection parts, when the dental prosthesis is found to be not fittable by pre-mounting before fixed at the cavity or the abutment tooth of a patient, an operation for restoring the dental prosthesis to be properly fittable can be easily carried out by grinding and finely adjusting these projection parts.

When the designing is completed, the dental prosthesis designed using the computer is produced. The dental prosthesis can be produced by cutting and grinding the block for a dental prosthesis by a cutting and grinding machine using the CAD/CAM system. Further, the dental prosthesis can be produced by photosensitively curing a photo-curable resin by laser irradiation from an optical shaping apparatus using the CAD system, where the photo-curable resin is photosensitively cured for every thin section of the dental prosthesis. Accordingly, the dental prosthesis can be easily produced. In order to fix the produced dental prosthesis to the cavity of a tooth or the abutment tooth, the dental prosthesis is pre-mounted in the cavity of a tooth or on the abutment as illustrated in Figs. 2 and 4 so as to check the fitting of it. When it is not fitted, the dental prosthesis can be easily adjusted. That is, since the portions contacted with the inner face of the cavity of a tooth or the outer face of the abutment tooth are only the projection parts P, or only the portion within the distance of 0.1 to 1 mm from the margin line and the projection parts P, only the projection parts P, which are not fitted, can be slightly removed so as to easily adjust the inclination and the fitting depth of the dental prosthesis. After the fitting of the dental prosthesis can be confirmed, the dental cement is coated in the cement space C, and the dental prosthesis is fixed at the cavity of a tooth or the abutment tooth.

## Claims

1. A dental prosthesis which is to be mounted in a cavity or covered on an abutment tooth,
wherein a cement space (C) is kept on a whole face opposite to the cavity or the abutment tooth except a plurality of projection parts (P) contacted with the cavity or the abutment tooth.

2. A dental prosthesis which is to be mounted in a cavity or covered on an abutment tooth,
wherein a cement space (C) is kept on a whole face opposite to the cavity or the abutment tooth except apluralityofprojectionparts (P) and a portion within a distance of 0.1 to 1 mm from a margin line.

3. The dental prosthesis as claimed in claim 1 or 2,
wherein the maximum thickness of the cement space (C) is 15 to 100 µm.

4. The dental prosthesis as claimed in any one of claims 1 to 3,
wherein the projection parts (P) are formed to have a streak shape being approximately parallel to a tooth axis.

5. The dental prosthesis as claimed in claim 4,
wherein a projection part (P) contacted with a bottom face of the cavity is further provided in the case of the dental prosthesis which is to be mounted in the cavity, or a projection part (P) contacted with a top part of the abutment tooth is further provided in the case of the dental prosthesis which is to be covered on the abutment tooth.

6. A method of designing a dental prosthesis, comprising steps of:
producing a gypsum model based on an impression taken from an oral cavity of a patient;
measuring a secondary gypsum model or a wax model produced from the gypsum model so as to obtain three-dimensional measuring data in the case of the dental prosthesis which is to be mounted in a cavity; or
measuring the gypsum model so as to obtain three-dimensional measuring data in the case of the dental prosthesis which is to be covered on the abutment tooth; and
designing the dental prosthesis which is to be mounted in the cavity or covered on the abutment tooth based on the obtained three-dimensional measuring data using a computer,
wherein a plurality of projection parts (P) contacted with the cavity or the abutment tooth is provided, and
wherein a cement space (C) is set on a whole face opposite to the cavity or the abutment tooth except the projection parts (P).

7. A method of designing a dental prosthesis, comprising steps of:
producing a gypsum model based on an impression taken from an oral cavity of a patient;
measuring a secondary gypsum model or a wax model produced from the gypsum model so as to obtain three-dimensional measuring data in the case of the dental prosthesis which is to be mounted in a cavity; or
measuring the gypsum model so as to obtain three-dimensional measuring data in the case of the dental prosthesis which is to be covered on the abutment tooth; and
designing the dental prosthesis which is to be mounted in the cavity or covered on the abutment tooth based on the obtained three-dimensional measuring data using a computer,
wherein a plurality of projection parts (P) contacted with the cavity or the abutment tooth and a portion within a distance of 0.1 to 1 mm from a margin line are provided, and
wherein a cement space (C) is set on a whole face opposite to the cavity or the abutment tooth except the projection parts (P) and the portion within the distance of 0.1 to 1 mm from the margin line.

8. The method of designing a dental prosthesis as claimed in claim 6 or 7,
wherein the maximum thickness of the cement space (C) is 15 to 100 µm.

9. The method of designing a dental prosthesis as claimed in any one of claims 6 to 8,
wherein the projection parts (P) are formed to have a streak shape being approximately parallel to a tooth axis.

10. The method of designing a dental prosthesis as claimed in claim 9,
wherein a projection part (P) contacted with a bottom face of the cavity is further provided in the case of the dental prosthesis which is to be mounted in the cavity, and a projection part (P) contacted with a top part of the abutment tooth is further provided in the case of the dental prosthesis which is to be covered on the abutment tooth.

11. A method of producing a dental prosthesis designed by the method of designing a dental prosthesis as claimed in any one of claims 6 to 10 by using a CAD/CAM system, the method comprising a step of cutting and grinding a block for a dental prosthesis by a cutting and grinding machine using the CAD/CAM system.

12. A method of producing a dental prosthesis designed by the designing method of a dental prosthesis as claimed in any one of claims 6 to 10 by using a CAD system, the method comprising a step of photosensitively curing a photo-curable resin by laser irradiation from an optical shaping apparatus using the CAD system,
wherein the photo-curable resin is photosensitively cured for every thin section of the dental prosthesis.
